(19) Europäisches Patentamt / European Patent Office / Office européen des brevets

(11) EP 2 953 923 B1

(12) # FASCICULE DE BREVET EUROPEEN

(45) Date de publication et mention de la délivrance du brevet:
**27.03.2019 Bulletin 2019/13**

(21) Numéro de dépôt: **14706906.6**

(22) Date de dépôt: **10.02.2014**

(51) Int Cl.:
***C07C 67/475*** *(2006.01)* ***C07C 253/30*** *(2006.01)*
***C07C 51/285*** *(2006.01)* ***C07C 255/23*** *(2006.01)*
***C07C 69/593*** *(2006.01)* ***C07C 55/18*** *(2006.01)*
***C07C 55/20*** *(2006.01)* ***C07C 51/09*** *(2006.01)*
***C07C 57/13*** *(2006.01)*

(86) Numéro de dépôt international:
**PCT/FR2014/050247**

(87) Numéro de publication internationale:
**WO 2014/122410 (14.08.2014 Gazette 2014/33)**

(54) **SYNTHESE CONJUGUEE D'UN NITRILE- ESTER/ACIDE ET D'UN DIESTER/DIACIDE**

KOMBINIERTE SYNTHESE EINES NITRILESTERS/EINER SÄURE UND EINES DIESTERS/EINER DISÄURE

COMBINED SYNTHESIS OF A NITRILE-ESTER/ACID AND OF A DIESTER/DIACID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.02.2013 FR 1351102**

(43) Date de publication de la demande:
**16.12.2015 Bulletin 2015/51**

(73) Titulaire: **Arkema France**
**92700 Colombes (FR)**

(72) Inventeurs:
- **DUBOIS, Jean-Luc**
  **F-69390 Millery (FR)**
- **COUTURIER, Jean-Luc**
  **F-69006 Lyon (FR)**
- **BRANDHORST, Markus**
  **F-69008 Lyon (FR)**

(74) Mandataire: **Hérard, Elise**
**ARKEMA France**
**Département Propriété Industrielle**
**420, rue d'Estienne d'Orves**
**92705 Colombes Cedex (FR)**

(56) Documents cités:
**WO-A1-2010/055273**
**WO-A1-2013/011226**
**FR-A1- 2 912 741**
**FR-A1- 2 941 694**
**FR-A1- 2 984 309**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 953 923 B1

## Description

DOMAINE DE L'INVENTION

La présente invention se rapporte à un procédé de synthèse conjuguée d'un nitrile-acide/ester et d'un diacide/diester (ou ses dérivés) de haute pureté à partir d'un acide/ester gras insaturé d'origine naturelle, ledit procédé comportant au moins une étape de métathèse croisée et une étape de coupure oxydante. L'invention a notamment pour objet la synthèse à la fois d'ω-aminoacide/ester et de diacide/diester, deux produits d'intérêt pour la synthèse de monomères de polymères, tels que les polyamides (PA) de spécialités.

ARRIERE PLAN TECHNIQUE

L'industrie des polyamides utilise toute une gamme de monomères, principalement des lactames, des ω-amino-acides, et des couples « diamine.diacide », désignés par le nombre de carbones par amide, contenus dans le motif de répétition du polyamide. On peut citer par exemple les polyamides : PA 6, PA 6.6, PA 6.10, PA 7, PA 8, PA 9, PA 11, PA 12, PA 13.
Ces monomères sont par exemple fabriqués par voie de synthèse chimique en utilisant notamment comme matière première des oléfines en C2 à C4, des cycloalcanes ou du benzène, mais aussi de l'huile de ricin (PA 11), de l'huile érucique ou lesquérolique (PA 13).
L'évolution actuelle en matière d'environnement conduit dans les domaines de l'énergie et de la chimie à privilégier l'exploitation des matières premières naturelles provenant d'une source renouvelable. C'est la raison pour laquelle certains travaux ont été repris pour élaborer sur le plan industriel des procédés utilisant des acides/esters gras comme matière première de fabrication de ces monomères.
Ce type d'approche n'a que peu d'exemples industriels. Un des rares exemples de procédé industriel utilisant un acide gras comme matière première est celui de la fabrication à partir de l'acide ricinoléique extrait de l'huile de ricin, de l'acide 11-aminoundécanoique, qui est à la base de la synthèse du polyamide 11 de marque Rilsan®. Ce procédé est décrit dans l'ouvrage « Les Procédés de Pétrochimie » de A. Chauvel et al. paru aux Editions TECHNIP (1986). L'acide 11-aminoundécanoique est obtenu en plusieurs étapes, méthanolyse de l'huile de ricin, pyrolyse du ricinoléate de méthyle pour obtenir l'undécylénate de méthyle qui est hydrolysé et l'acide formé est soumis à une hydrobromuration pour donner l'acide ω-bromé d'où l'on passe par amination à l'acide 11-aminoundécanoïque.

Les principaux travaux de recherche ont porté sur la synthèse de l'acide 9-aminononanoïque, précurseur du PA 9, à partir de l'acide oléique d'origine naturelle.

En ce qui concerne ce monomère particulier, on peut citer l'ouvrage « n-Nylons, Their Synthesis, Structure and Properties » - 1997 Ed.J. Wiley et Sons dont le chapitre 2.9 (pages 381 à 389) est consacré au polyamide 9. Cet article donne la synthèse des réalisations et des travaux conduits sur le sujet. On y mentionne, page 381, le procédé développé par l'ex Union Soviétique ayant conduit à la commercialisation du Pelargon®. On y mentionne également, page 384, un procédé développé au Japon utilisant l'acide oléique venant de l'huile de soja comme matière première. La description correspondante fait référence à l'ouvrage de A. Ravve « Organic Chemistry of Macromolecules » (1967) Marcel Dekker, Inc., dont la partie 15 est consacrée aux polyamides et qui mentionne en page 279 l'existence d'un tel procédé.

Pour être complet sur l'état de l'art en la matière, il faut citer les nombreux articles publiés par E. H. Pryde et al. entre 1962 et 1975 dans - Journal of the American Oil Chemists Society - « Aldehydic Materials by the Ozonization of Vegetable Oils » Vol. 39 pages 496-500 ; « Pilot Run, Plant Design and Cost Analysis for Reductive Ozonolysis of Methyl Soyate » Vol. 49 pages 643-648 et R.B. Perkins et al. « Nylon-9 from Unsaturated Fatty Derivatives : Preparation and Characterization » JAOCS, Vol. 52 pages 473-477. Il est à noter que le premier de ces articles fait aussi référence page 498 à des travaux antérieurs réalisés par des Japonais : H. Otsuki et H. Funahashi.

Pour résumer cette partie de l'état de l'art visant ce type de synthèse du PA 9 à partir d'huiles végétales, on peut décrire le mécanisme réactionnel simplifié suivant appliqué à l'ester oléique, extrait des huiles par méthanolyse :

Ozonolyse réductrice :

$$H_3C-(CH_2)_7-CH=CH-(CH_2)_7-COOCH_3 + (O_3, H_2) \rightarrow H_3C-(CH_2)_7-CHO + OHC-(CH_2)_7-COOCH_3$$

Amination réductrice :

$$OHC-(CH_2)_7-COOCH_3 + (NH_3, H_2) \rightarrow H_2N-(CH_2)_8-COOCH_3 + H_2O$$

suivie d'une hydrolyse conduisant à l'amino-acide.

Cette voie très séduisante sur le plan réactionnel présente cependant un inconvénient économique important

constitué par la production lors de la première étape de l'aldéhyde pélargonique très difficile à valoriser, y-compris dans l'industrie des polyamides.

Le document de brevet GB741739 décrit pour sa part la synthèse de ce même acide à partir de l'acide oléique mais en utilisant la voie oléonitrile. Une voie analogue est citée dans l'article de R.B. Perkins et al cité ci-dessus p. 475.

Le schéma réactionnel simplifié de ce procédé est le suivant.

$$H_3C\text{-}(CH_2)_7\text{-}CH\text{=}CH\text{-}(CH_2)_7\text{-}COOH + NH_3 \rightarrow H_3C\text{-}(CH_2)_7\text{-}CH\text{=}CH\text{-}(CH_2)_7\text{-}CN + 2\ H_2O$$

$$H_3C\text{-}(CH_2)_7\text{-}CH\text{=}CH\text{-}(CH_2)_7\text{-}CN + (O_3 + H_2O) \rightarrow H_3C\text{-}(CH_2)_7\text{-}COOH + CN\text{-}(CH_2)_7\text{-}COOH$$

$$CN\text{-}(CH_2)_7\text{-}COOH + 2\ H_2 \rightarrow H_2N\text{-}(CH_2)_8\text{-}COOH$$

Cette synthèse conduit à l'acide pélargonique (ou nonanoïque) $H_3C\text{-}(CH_2)_7\text{-}COOH$ comme sous-produit.

La demanderesse a pour sa part développé des procédés de fabrication de tels monomères ω-aminoacides qui utilisent notamment les réactions de métathèse. On peut citer à ce sujet les demandes de brevet (WO08104722, WO10055273, WO10089512) qui décrivent des procédés dans lesquels la fonction amine terminale de l'ω-aminoacide résulte de l'hydrogénation d'une fonction nitrile, introduite soit par métathèse croisée avec l'acrylonitrile (WO08104722) soit par ammoniation de la fonction acide (WO10055273 et WO10089512) dans le cadre d'un procédé transitant par un nitrile ω-insaturé ou un dinitrile mono-insaturé.

Les diacides sont quant à eux obtenus industriellement selon différentes méthodes, mais qui toutes présentent certains inconvénients. Un large panorama de ces méthodes est développé dans l'Encyclopédie Kirk-Othmer, 4ième Edition, Vol. A8 pages 118-136. On peut y distinguer les méthodes par dégradation telles que l'ozonolyse ou l'oxydation des acides gras insaturés végétaux.

L'ozonolyse de l'acide oléique, de l'acide pétrosélinique et de l'acide érucique permet de produire respectivement les diacides à 9, 6 et 13 atomes de carbone.

Un autre exemple est le clivage de l'acide ricinoléique par action de la soude à une température supérieure à 180°C. Cette méthode utilisée industriellement permet d'obtenir le diacide à 10 atomes de carbone. La même méthode appliquée à l'acide lesquérolique conduit à la formation d'un diacide à 12 atomes de carbone.

Cette méthode présente l'avantage d'utiliser des matières premières renouvelables mais est limitée essentiellement à la fabrication du diacide en C10, l'acide lesquérolique étant encore peu répandu, et donc cette méthode peu utilisée.

Il est également possible d'obtenir des diacides à partir de molécules de plus petites tailles en utilisant des techniques variantes de la carbonylation.

On peut citer enfin la fermentation bactérienne de paraffines, méthode bien connue et qui permet d'obtenir de nombreux diacides de longueur de chaîne variable. Cependant cette méthode ne permet pas d'obtenir des diacides de longueur de chaine supérieure à 16 atomes de carbone car les paraffines ont alors un point de fusion beaucoup trop élevé pour pouvoir être transformées. Un autre inconvénient important est que les bactéries consomment une partie des paraffines pour assurer leur croissance, conduisant à des rendements faibles et à la nécessité de purifier les produits.

L'ozonolyse de l'acide érucique et de l'acide oléique est utilisée industriellement pour la production des diacides à respectivement 13 et 9 atomes de carbone. Cette technique présente plusieurs inconvénients. En effet, dans le cas de la production du diacide à 9 atomes de carbone, la matière première est l'acide oléique. Celui-ci est présent dans la nature en mélange avec de l'acide stéarique (acide de même longueur de chaine mais saturé). L'acide stéarique ne peut pas réagir lors de l'ozonolyse. Il a cependant un point d'ébullition proche du diacide à 9 atomes de carbones, ce qui complexifie sa séparation. Il faut donc soit utiliser un acide oléique très pur (et par conséquent plus cher) soit utiliser des techniques de séparation efficaces mais onéreuses en aval du procédé de coupure oxydante pour isoler le diacide.

D'autre part, ces procédés de coupure oxydante génèrent en coproduit de l'acide pélargonique - acide linéaire saturé à 9 atomes de carbone - qui s'adresse à un marché (lubrifiants, herbicides...) très différent de celui des polymères, alors que les diacides s'adressent aux marchés des polyamides, polyesters et solvants (esters). Dans ce type de procédé, produits et coproduits ont des marchés de croissances différentes. Et comme l'un des produits a une valeur de croissance faible ou même de décroissance, la valeur économique du procédé et donc celle de l'autre produit est en large partie réduite. Il est donc avantageux lorsqu'un produit est obtenu avec un coproduit de trouver des applications s'adressant aux mêmes marchés.

La demanderesse a pour sa part lancé des travaux dans ce domaine qui utilisent notamment les réactions de métathèse dans le cadre d'un processus multi-réactionnel.

Le document de brevet (WO08155506) décrit un procédé consistant dans une première étape à transformer par pyrolyse ou éthénolyse l'ester gras insaturé en ester gras ω-insaturé puis dans une deuxième étape à soumettre le produit ainsi obtenu à une réaction de métathèse, soit homométathèse pour obtenir un composé de formule $ROOC\text{-}(CH_2)_m\text{-}CH\text{=}CH\text{-}(CH_2)_m\text{-}COOR$, soit métathèse croisée avec un composé de formule $R_2OOC\text{-}(CH_2)_r\text{-}CH\text{=}CH\text{-}R_3$ pour obtenir un composé insaturé de formule $ROOC\text{-}(CH_2)_m\text{-}CH\text{=}CH\text{-}(CH_2)_r\text{-}COOR_2$, qui seront transformés par

hydrogénation en composés saturés.

Le document de brevet (WO09047444) décrit un procédé de synthèse de diacides ou de diesters à partir d'esters gras naturels à longue chaîne mono-insaturés consistant dans une première étape, à oxyder par fermentation l'ester gras naturel en un diester carboxylique mono-insaturé puis dans une deuxième étape à soumettre le produit de la première étape à une métathèse croisée avec un composé de formule $R_2OOC-(CH_2)_x-CH=CH-R_3$ pour obtenir un composé insaturé de formule $ROOC-(CH_2)_q-CH=CH-(CH_2)_x-COOR_2$, pouvant par hydrogénation conduire au composé saturé.

De manière connue, la métathèse croisée consiste à faire réagir en présence d'un catalyseur deux molécules insaturées selon le processus réactionnel schématique suivant :

$$A_1A_2 - C{=}C - B_1B_2 + D_1D_2 - C{=}C - E_1E_2 \Leftrightarrow$$

$$A_1A_2 - C{=}C - D_1D_2 + A_1A_2 - C{=}C - E_1E_2 + B_1B_2 - C{=}C - D_1D_2 + B_1B_2 - C{=}C - E_1E_2$$

Une telle réaction équilibrée pose naturellement des problèmes en vue d'une utilisation industrielle. La présence de quatre produits de la réaction lorsqu'on ajoute les deux composés réactionnels qui, par hypothèse, n'ont pas réagi totalement contribue à la constitution d'un mélange complexe.

L'homme du métier cherche toujours à influencer la composition du mélange réactionnel résultant en choisissant soigneusement les quantités respectives des deux réactifs ainsi que la structure du réactif $D_1D_2$ - C=C- $E_1E_2$ afin que les produits formés soient aisément séparables du milieu, et notamment séparables sous forme gazeuse, ce qui présente en outre l'avantage de déplacer l'équilibre de la réaction et ainsi d'augmenter les quantités de produits de la réaction. La difficulté est de sélectionner des réactifs qui n'interfèrent pas avec les produits, et qui ne génèrent pas de coproduits difficiles à séparer des produits de la réaction.

Il faut cependant noter que la réaction est effectuée en présence d'un catalyseur qui est également actif pour les réactions d'homo-métathèse. Cela signifie qu'outre les réactions citées ci-dessus on est également en présence de la réaction ci-dessous :

$$A_1A_2 - C{=}C - B_1B_2 + D_1D_2 - C{=}C - E_1E_2 \Leftrightarrow$$

$$A_1A_2 - C{=}C - A_1A_2 + B_1B_2 - C{=}C - B_1B_2 + D_1D_2 - C{=}C - D_1D_2 + E_1E_2 - C{=}C - E_1E_2$$

Cela implique que lors d'une métathèse croisée, l'opérateur est en présence d'un milieu réactionnel comportant dix produits potentiels. Pour limiter le nombre de produits potentiels de métathèse, il est dans certains cas possible de limiter la formation de certains produits secondaires et/ou de limiter la faisabilité chimique de certaines réactions intervenant au cours de la métathèse. Ceci peut se faire notamment par le choix du co-réactif et/ou par une séparation de certains composés au fur et à mesure de leur formation. Par exemple, en sélectionnant des composés ayant une double liaison terminale ou proche de l'extrémité de chaine, les coproduits de la réaction seront des oléfines légères (gazeuses) qui s'élimineront spontanément du milieu réactionnel liquide.

A ces obstacles inhérents à la réaction proprement dite, vient s'ajouter une difficulté liée au catalyseur qui, outre son activité dans la métathèse, est également actif en isomérisation. Il a été observé, dans certains cas, que cette activité isomérisante du catalyseur augmente lorsque son activité décroit en métathèse. Le catalyseur de métathèse désactivé pour la réaction de métathèse semble se transformer en une espèce active pour catalyser les réactions d'isomérisation. Dans ces conditions, le catalyseur « en phase d'épuisement » en présence des oléfines à longues chaines de la charge ou des produits entraine un déplacement de la double liaison du réactif et/ou produit acide gras. Ceci conduit à des mélanges d'isomères et entraîne la formation d'un mélange de produits présentant différentes longueurs de chaine. Ces dernières étant cependant très proches, leurs séparations respectives sont particulièrement difficiles.

Lorsque la métathèse croisée est utilisée avec pour objectif de synthétiser un produit cible, par exemple $A_1A_2$ - C=C - $D_1D_2$ dans l'équation précédente, ce dernier sera donc accompagné le cas échéant des trois autres composés de la métathèse croisée ainsi que de certains composés issus de l'homométathèse, réaction qui accompagne automatiquement la réaction principale, mais aussi de mélanges d'isomères de ces composés.

La présente invention a pour but la fabrication de produits « purs » plutôt que celle de mélanges, et a donc pour but de fournir un procédé dans lequel le rendement en produit cible est maximisé et dans lequel le (ou les) coproduit(s) synthétisé(s) présentent une valeur ajoutée substantielle et un degré de pureté suffisant sans qu'il soit nécessaire d'appliquer des techniques de purifications sophistiquées et donc chères. La présente invention a également

pour but de fournir un procédé dans lequel produit et coproduit s'adressent de préférence au même marché final.

La Demanderesse a maintenant trouvé un tel procédé permettant de pallier les inconvénients précédents et de produire à la fois un ω-aminoacide comme produit principal et un diester/diacide à longue chaine pur comme co-produit, ou ses produits dérivés bi-fonctionnels tels que les acide(ester)-alcools, tous permettant de fabriquer directement des monomères pour l'industrie des polymères. Le procédé de l'invention permet ainsi de disposer sur le même site de deux matières premières utilisables dans un même but, à savoir la fabrication de polymères, et encore mieux dans un même type de procédé de polymérisation.

Sans vouloir être liée par une quelconque théorie, la demanderesse pense que le catalyseur de métathèse voit son activité isomérisante décroitre lors de la réaction d'homo-métathèse de l'acide gras si la réaction d'homo-métathèse est réalisée concomitamment avec une métathèse croisée en présence d'un co-réactif nitrile insaturé, ce qui permet d'arriver au résultat surprenant obtenu par le procédé de l'invention.

DESCRIPTION DE MODES DE REALISATION DE L'INVENTION

L'invention a donc pour objet un procédé de synthèse conjuguée d'un nitrile-ester(acide) mono-insaturé (III) et d'un composé carbonylé bi-fonctionnel (V), symétrique ou non, de formule $R_2$-$(CH_2)_n$-COR' dans laquelle R' est H ou OH, à partir d'un (même) composé gras insaturé (I) comportant au moins une fonction, respectivement ester ou acide de formule,

$$R_1\text{-CH=CH-}[(CH_2)_q\text{-CH=CH}]_p\text{-}(CH_2)_n\text{-}R_2 \qquad \text{(I)}$$

dans laquelle : $R_1$ est H ou un radical alkyle comprenant de 1 à 4 atomes de carbone, $R_2$ est une fonction ester ou acide et n, p et q sont des entiers tels que $3 \leq n \leq 13$ ; $0 \leq p \leq 2$ ; $0 \leq q \leq 1$
caractérisé en ce qu'il comprend :

- une étape de métathèse croisée mc1 du composé (I) avec un composé nitrile insaturé (II) choisi parmi l'acrylonitrile, le fumaronitrile, le 2-butènenitrile, le 2-pentènenitrile, le 3-pentène nitrile, le 4-pentènenitrile,
  dans laquelle mc1 est menée à conversion partielle de façon à obtenir, et récupérer séparément, au moins les produits suivants: un nitrile-acide(ester) mono-insaturé (III) et un composé (IV) symétrique, respectivement diester ou diacide comportant une double liaison située au milieu de la chaîne moléculaire du composé (IV) de formule $R_2$-$(CH_2)_n$-HC=CH-$(CH_2)_n$-$R_2$ résultant de l'homo-métathèse du composé (I),
  puis,

- une étape de coupure par oxydation cp2 de la double liaison respectivement du composé (IV) diester ou diacide, de façon à former un seul type de composé carbonylé (V) de formule $R_2$-$(CH_2)_n$-COR' dans laquelle R' est H ou OH, selon les conditions opératoires choisies pour la coupure par oxydation cp2.

Au sens de l'invention, on entend par « synthèse conjuguée » de 2 produits, la synthèse à la fois, au cours du procédé de l'invention, de 2 produits cibles parfois nommés ici le produit et le coproduit, à savoir le composé III d'une part et le composé V d'autre part.

Au sens de l'invention, par produit ou coproduit :

- « pur » on entend un produit ne contenant que peu d'impuretés, typiquement moins de 15% en poids, de préférence moins de 10%, de préférence moins de 5%, plus préférablement moins de 2%, ou mieux moins de 1% en poids, sur le poids de produit ou de coproduit ;

- « à longue chaine » on entend ici un produit dont la chaine principale comprend au moins 8 atomes de carbone, de préférence de 8 à 20 carbones, de préférence de 9 à 18 carbones, de préférence de 9 à 14 carbones, de préférence 9, 10 ou 13 atomes de carbone pour le diacide/diester final, de préférence 11 ou 12 ou 13 atomes de carbone pour le nitrile-acide/ester ou l'aminoacide/aminoester final.

Par composé « gras » au sens de l'invention, on entend un composé qui comporte de préférence de 10 à 24 atomes de carbone, de préférence de 14 à 24 atomes de carbone par molécule.

Le composé gras insaturé (I) de formule $R_1$-CH=CH-$[(CH_2)_q$-CH=CH$]_{p-}(CH_2)_n$-$R_2$ est avantageusement choisi parmi les acides gras ou les esters d'acides gras, ces esters pouvant être des esters de mono-alcool, de diol ou de triol, y-compris les mono-, di-, et tri-glicérides, d'origine végétale ou animale incluant ceux issus d'algues naturelles.

Le composé I comporte au moins une insaturation, c'est-à-dire une double liaison C=C. L'insaturation est localisée en position x par rapport au groupement acide ou ester : cette position est désignée par convention « delta

x »). Ceci permet de déterminer la formule de l'ω-aminoacide final.

On peut citer à titre d'exemples d'acides gras, les acides en C10, les acides obtusilique (cis-4-décénoïque) et caproléique (9-décénoïque), les acides en C12, les acides lauroléique (cis-5-dodécénoïque) et lindérique (cis-4-dodécénoïque), les acides en C14, les acides myristoléique (cis-9-tétradécénoïque), physétérique (cis-5-tetradécénoïque) et tsuzuique (cis-4-tetradécénoïque), l'acide en C16, l'acide palmitoléique (cis-9-hexadécénoïque), les acides en C18, les acides oléique (cis-9-octadécénoïque), élaidique (trans-9-octadécénoïque), pétrosélinique (cis-6-octadécénoïque), vaccénique (cis-11-octadécénoïque) et ricinoléique (12-hydroxy--cis-9-octadécénoïque), les acides en C20, les acides gadoléique (cis-9-eicosénoïque), gondoïque (cis-11-eicosénoïque), l'acide cis-5-eicosènoïque et l'acide lesquérolique (14-hydroxy-cis-11-eicosénoïque), les acides en C22, les acides cétoléique (cis-11-docosénoïque) et érucique (cis-13-docosénoïque) ainsi que les acides polyinsaturés linoléique et linolénique.

Ces divers acides sont issus des huiles végétales extraites de diverses plantes oléagineuses telles que le tournesol, le colza, le ricin, le lesquerella, l'olive, le soja, le palmier, l'avocat, l'argousier, la coriandre, le céleri, l'aneth, la carotte, le fenouil, le Limnanthes Alba (meadowfoam), le carthame, la caméline, le Jatropha. Ils sont issus également du monde animal terrestre ou marin, et dans ce dernier cas, aussi bien sous forme de poissons, de mammifères que d'algues. Il s'agit en général de graisses provenant de ruminants, de poissons comme la morue, ou de mammifères marins comme les baleines ou les dauphins.

Pour des raisons techniques de conduite de la réaction de métathèse, il est souvent préférable de modifier cet ester/acide gras en le soumettant à une réaction préalable comprenant une éthénolyse, buténolyse ou propénolyse ou un craquage thermique (pyrolyse) permettant de conduire à un acide(ester) gras de formules $CH_2=CH-(CH_2)_n-R_2$, $CH_3-CH=CH-(CH_2)_n-R_2$ ou $CH_3-CH_2-CH=CH-(CH_2)_n-R_2$.

De façon encore plus préférée le composé I est un acide ou ester gras de formule $CH_2=CH-(CH_2)_n-COOR_3$ dans laquelle

$R_3$ est H ou un radical alkyle comprenant de 1 à 11 atomes de carbone et,

n est un nombre entier compris dans la gamme de 3 à 13.

Le composé nitrile insaturé (II) est avantageusement choisi parmi l'acrylonitrile, le fumaronitrile, le 2-butènenitrile, le 2-pentènenitrile, le 3-pentène nitrile, le 4-pentènenitrile, et leurs mélanges.

L'étape de métathèse croisée est conduite de préférence avec l'acrylonitrile pour composé nitrile (II). En effet l'acrylontrile est largement disponible, peu onéreux et c'est en particulier le seul nitrile qui puisse donner de l'acide 11-aminoundécanoïque à partir du décénoate de méthyle. Un autre avantage est que le coproduit de la réaction est l'oléfine la plus légère qui soit : l'éthylène.

L'étape de métathèse croisée selon le procédé de l'invention est menée à conversion partielle de façon à obtenir et récupérer séparément les composés III et IV.

Avantageusement, l'étape de métathèse croisée mc1 est menée à un taux de conversion compris dans la gamme de 30 % à 99 %, de préférence de 30 à 90%, de préférence de 40 à 90 %. Le taux de conversion est réglé par les conditions opératoires et notamment la durée de la réaction et les moyens mis en oeuvre pour l'extraction du composé gazeux formé lors de la réaction de métathèse. De plus, il est avantageux de limiter ce taux de conversion afin de préserver les performances du catalyseur.

Avantageusement, le procédé de l'invention comprend en outre une étape d'hydrogénation du nitrile-ester/acide mono-insaturé (III) obtenu pour former un α,ω-amino-ester/acide saturé.

Avantageusement, le procédé comprend en outre, dans le cas où (IV) est un diester, une étape optionnelle intermédiaire d'hydrolyse entre mc1 et cp2 pour obtenir un composé (IV) diacide.

La réaction de métathèse est conduite en présence d'au moins un catalyseur de métathèse. Ces catalyseurs sont bien connus et il en existe toute une gamme. On peut citer par exemple les complexes au tungstène développés par Schrock et al (J. Am. Chem. Soc. 108:2771, 1986) ou Basset et al. (Angew. Chem., Ed. Engl. 31:628, 1992). Plus récemment, sont apparus les catalyseurs dits de Grubbs (voir Grubbs et al., Angew. Chem., Ed. Engl. 34 :2039, 1995 et Organic Letters 1:953, 1999) qui sont des complexes ruthénium-benzylidène opérant en catalyse homogène. D'autres travaux ont été conduits pour la réalisation de catalyseurs immobilisés, c'est-à-dire de catalyseurs dont le principe actif est celui du catalyseur homogène, notamment les complexes ruthénium-carbène, immobilisés sur un support inactif.

Le procédé selon l'invention utilise avantageusement au moins un catalyseur de métathèse de type ruthénium-carbène. Ledit catalyseur ruthénium-carbène est choisi de préférence parmi les catalyseurs chargés ou non-chargés de formule générale :

$$(X_1)_a (X_2)_b Ru(\text{carbène } C) (L_1)_c (L_2)_d (L_3)_e$$

dans laquelle :

- a, b, c, d et e sont des nombres entiers, identiques ou différents, avec a et b égaux à 0, 1 ou 2 ; c, d et e égaux à 0, 1, 2, 3 ou 4;
- $X_1$ et $X_2$, identiques ou différents, représentent chacun un ligand mono- ou multi-chélatant, chargé ou non ; à titre d'exemples, on pourra citer les halogénures, le sulfate, le carbonate, les carboxylates, les alcoolates, les phénolates, les amidures, le tosylate, l'hexafluorophosphate, le tétrafluoroborate, le bis-triflylamidure, un alkyle, le tétraphénylborate et dérivés ; $X_1$ ou $X_2$ peuvent être liés à $L_1$ ou $L_2$ ou au carbène C de façon à former un ligand bidenté ou chélate sur le ruthénium ; et
- $L_1$, $L_2$ et $L_3$ identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéther, ou un carbène hétérocyclique ; $L_1$, $L_2$ ou $L_3$ peuvent être liés au carbène C de façon à former un ligand bidenté ou chélate, ou tridenté.

Le carbène C est représenté par la formule générale : $CR_1R_2$ pour laquelle $R_1$ et $R_2$ sont des groupes identiques ou différents tels que l'hydrogène ou tout autre groupe hydrocarboné, fonctionnalisé ou non, de type saturé, insaturé, cyclique, aromatique, branché et/ou linéaire. A titre d'exemples, on pourra citer les complexes du ruthénium alkylidènes, benzylidène, benzylidène éther, ou cumylènes tels que les vinylidènes Ru=C=CHR ou allénylidènes $Ru=C=C=CR_1R_2$ ou indénylidènes.

Un groupe fonctionnel (permettant d'améliorer la rétention du complexe du ruthénium dans un liquide ionique) peut être greffé sur au moins l'un des ligands $X_1$, $X_2$, $L_1$, $L_2$, ou sur le carbène C. Ce groupe fonctionnel peut être chargé ou non chargé tel que de préférence un ester, un éther, un thiol, un acide, un alcool, une amine, un hétérocycle azoté, un sulfonate, un carboxylate, un ammonium quaternaire, un guanidinium, un phosphonium quaternaire, un pyridinium, un imidazolium, un morpholinium ou un sulfonium.

Le catalyseur de métathèse peut être éventuellement hétérogénéisé sur un support afin de faciliter sa récupération/recyclage.

Les catalyseurs de métathèse croisée du procédé de l'invention sont de préférence des carbènes du ruthénium décrits par exemple dans Aldrichimica Acta, vol 40, n°2, 2007, p.45-52.

Des exemples de tels catalyseurs sont les catalyseurs de Grubbs, les catalyseurs Hoveyda-Grubbs, les catalyseurs Piers-Grubbs, et autres catalyseurs de métathèse du même type, qu'ils soient dits de « 1ère génération », « 2ème génération » ou de « 3ème génération ».

Les catalyseurs de Grubbs sont basés sur un atome de ruthénium entouré par 5 ligands :

- 2 ligands anioniques, tels que des halogénures ;
- 2 ligands donneurs d'électrons, tels que les tri-alkyl-phosphines, ou les carbènes N-hétérocycliques saturés (appelés ligands NHC) ;
- un groupement alkylidène, tels que des groupements méthylènes $=CR_2$ substitués ou non.

On classe ces catalyseurs de métathèse en deux catégories, suivant la nature de leurs ligands L donneurs d'électrons :

- ceux qui contiennent deux ligands phosphine (et pas de ligand NHC saturé), développés en premier, sont des catalyseurs de type 1ère génération ;
- ceux qui contiennent un ligand NHC saturé (carbène hétérocyclique) sont des catalyseurs de type 2ème génération.

Un type de catalyseur dit « Hoveyda-Grubbs » contient parmi les ligands donneurs d'électrons, un ligand chélatant benzylidène-éther, et soit une phosphine (1ère génération) soit un ligand NHC saturé (2ème génération), le plus souvent substitué par des phényles généralement substitués par des groupements mésityles (Mes) ou bien par des groupements isopropyles (iPr).

Un autre type de catalyseur dit « Piers-Grubbs », forme un complexe cationique à quatre ligands qui ne nécessite pas la dissociation d'un ligand avant la réaction.

D'autres types de catalyseurs sont les catalyseurs « Umicore », « Zanan », « Grela ».

De manière générale, le choix du catalyseur dépend de la réaction considérée.

Selon un mode de réalisation avantageux, le catalyseur est dépourvu de phosphine.

Des catalyseurs préférés sont les catalyseurs suivants :

(1) Le catalyseur désigné par « Hoveyda-Grubbs 2 », de formule suivante :

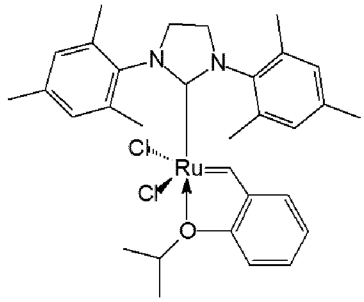

(2) Le catalyseur désigné par « M51 », de formule suivante :

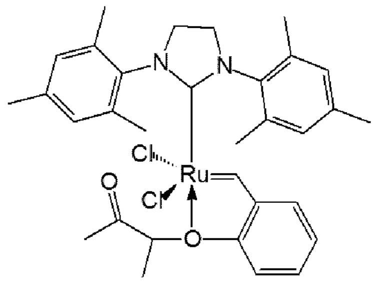

(3) Le catalyseur désigné par « M71-SIPr », de formule suivante :

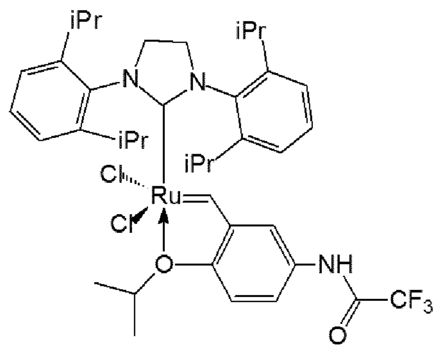

(4) Le catalyseur désigné par « M71-SIMes », de formule suivante :

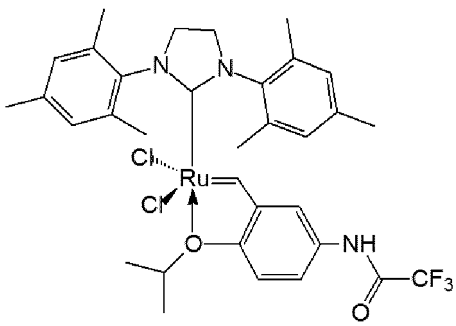

(4) Le catalyseur désigné par « M72-SIPr », de formule suivante :

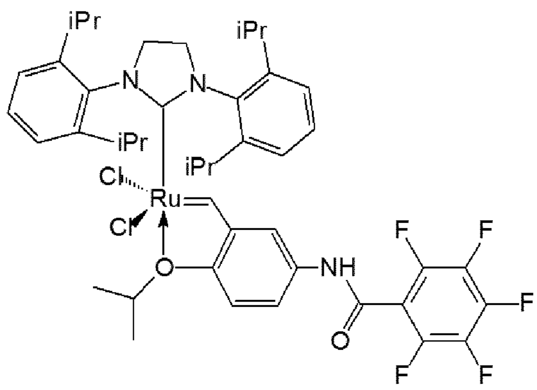

(5) Le catalyseur désigné par « M73-SIPr », de formule suivante :

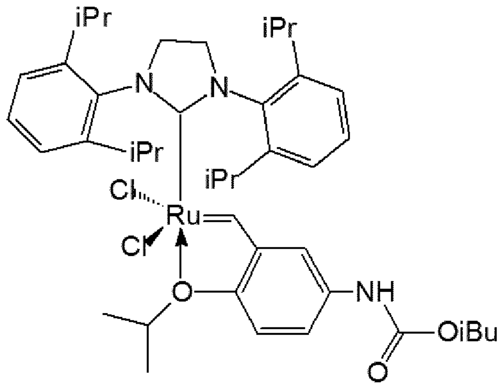

(6) Le catalyseur désigné par « M74-SIPr », de formule suivante :

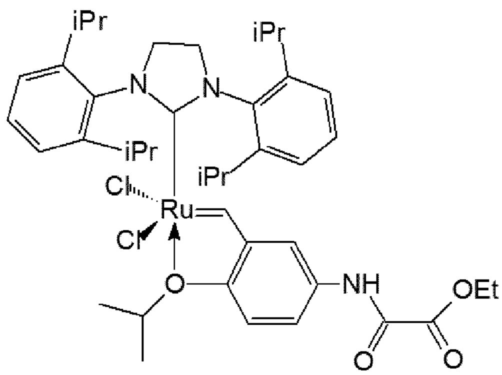

(7) Le catalyseur désigné par « Nitro-Grela-SIMes », de formule suivante :

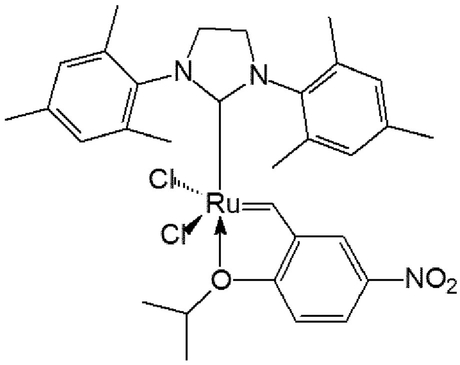

(8) Le catalyseur désigné par « Nitro-Grela-SIPr », de formule suivante :

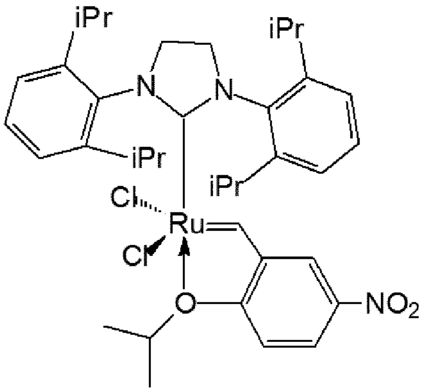

(9) Le catalyseur désigné par « Apeiron AS2034 », de formule suivante :

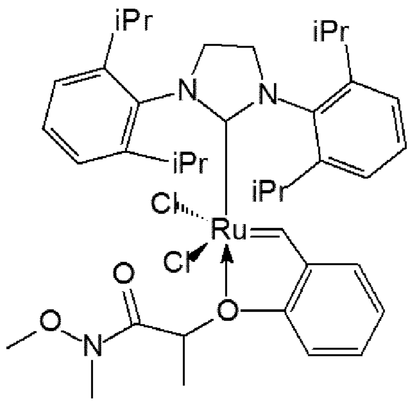

(10) Le catalyseur désigné par « Zannan 44-0082 (Strem) », de formule suivante :

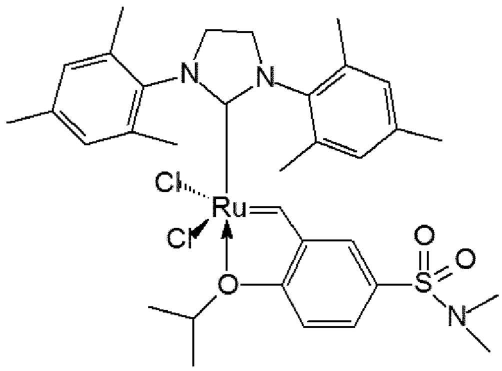

(11) Le catalyseur désigné par « M831-SIPr », de formule suivante :

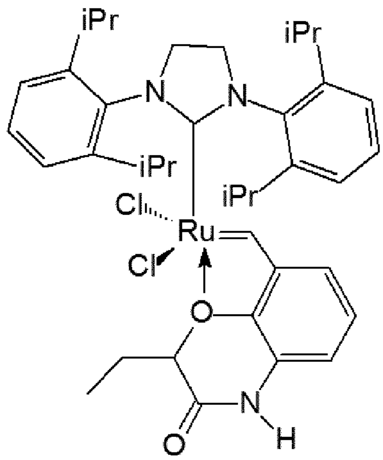

(12) Le catalyseur désigné par « M832-SIPr », de formule suivante :

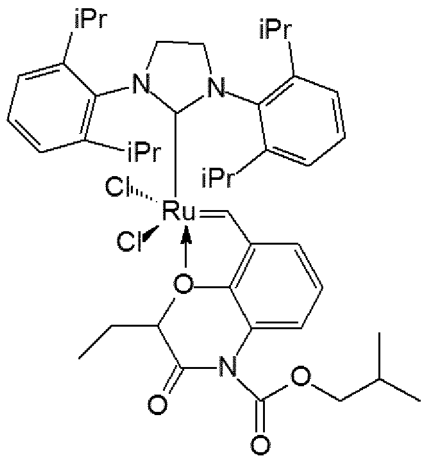

(13) Le catalyseur désigné par « M853-SIPr », de formule suivante :

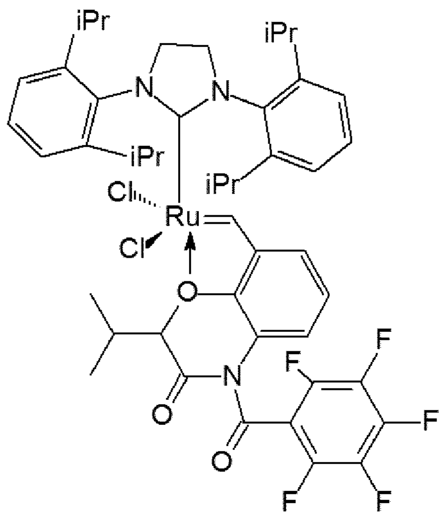

(14) Le catalyseur désigné par « M863-SIPr », de formule suivante :

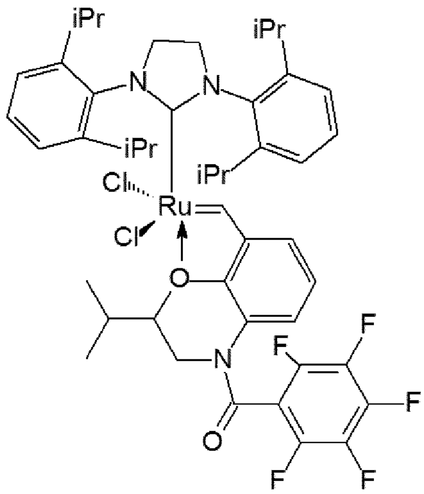

(15) Le catalyseur désigné par « Materia C711 », de formule suivante :

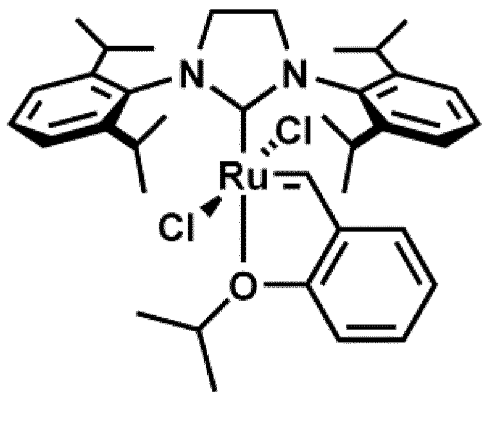

La réaction de métathèse est conduite de préférence en milieu liquide dans les conditions opératoires suivantes.
La température est généralement comprise dans la gamme de 20 à 160°C et de préférence dans la gamme de 20 à 120°C.
La pression est généralement comprise dans la gamme de 1 à 30 bars. La réaction sera de préférence conduite à basse pression comprise dans la gamme de 1 à 10 bars et de façon plus préférée à la pression atmosphérique lorsque

la température d'ébullition des réactifs mis en jeu le permet. En effet, si l'on vise toujours un dégagement d'oléfine légère, éthylène ou autre, il est avantageux de travailler à basse pression, pression atmosphérique de préférence. Par exemple, un composé tel que l'acrylonitrile en C3 dont le point de d'ébullition est de l'ordre de 80 °C permet de travailler à pression atmosphérique.

La réaction peut être conduite sans solvant ou en présence d'au moins un solvant, tel que le toluène, les xylènes ou le dichlorométhane par exemple. La réaction est de préférence conduite sans solvant.

Le composé diester ou diacide insaturé symétrique (IV) est soumis dans le procédé de l'invention à une coupure oxydante ou coupure par oxydation de la double liaison, qui conduit à la formation d'un seul type de composé V carbonylé.

La réaction de coupure oxydante est une réaction bien connue. Le procédé de l'invention utilise au cours de l'étape cp2 une ou plusieurs types de réactions de coupure oxydante, tels que ceux décrits ci-après.

Selon un premier mode de réalisation, la coupure est réalisée au moyen d'un oxydant fort tel que $KMnO_4$ sous forme concentrée et à la chaleur (température comprise dans la gamme de 20 et 150°C), comme décrit par exemple dans « Organic Chemistry » de L.G. Wade Jr. 5th Edition Chapter 8 Reactions of Alkenes.

Selon un deuxième mode de réalisation, la coupure est réalisée au moyen d'un oxydant fort tel que le chlorochromate d'ammonium. L'article de G.S. Zhang et al. dans Chinese Chemical Letters, Vol 5, N°2, pp105-108 de 1994, décrit la coupure oxydante à partir du diol correspondant à l'acide oléique (voir entrée 29 du tableau), cette coupure oxydante est effectuée en utilisant le chlorochromate d'ammonium comme oxydant. Le diol quant à lui, est obtenu par époxydation de l'acide oléique suivie d'une hydrolyse du pont époxy.

Selon encore un troisième mode de réalisation, la coupure est réalisée par irradiation. L'article de F. Drawert et al. dans Chem. Mikrobiol. Technol. Lebensm. 1, 158-159 (1972) décrit une voie de coupure oxydante par irradiation de l'huile de tournesol.

Le plus souvent, ces coupures avec oxydant fort conduisent à la formation de composés de type acide.

Selon un autre mode préféré de réalisation, la coupure oxydante cp2 est réalisée en utilisant comme oxydant l'eau oxygénée et/ou l'ozone.

Ces voies sont notamment exposées dans le document de brevet GB743491 qui illustre les mécanismes des réactions faisant intervenir l'eau oxygénée et/ou l'ozone, avec la formation des ozonides sur la coupure de la double liaison. Le document de brevet US2813113 décrit un procédé d'ozonolyse oxydante d'un acide gras tel que l'acide oléique qui consiste dans une première étape à traiter l'acide par de l'oxygène associé à de l'ozone pour former des ozonides puis dans une deuxième étape à oxyder ces derniers par l'oxygène. De nombreux travaux ont été conduits sur l'utilisation de l'ozone comme agent oxydant. La coupure oxydante de l'acide oléique en acides pélargonique et azélaique est la plus importante application industrielle de l'ozonolyse.

Le produit final obtenu dépend des conditions dans lesquelles la réaction d'oxydation par ozonolyse ou avec l'eau oxygénée est conduite. Dans le cas d'une ozonolyse conduite en milieu oxydant (en présence d'oxygène), le processus d'oxydation se poursuit jusqu'à la formation des fonctions acides ; on parle alors d'ozonolyse oxydante. En revanche, si on conduit l'oxydation dans des conditions réductrices, par décomposition de l'ozonide en présence d'hydrogène par exemple ou en présence de composés bloquant le processus d'oxydation, l'oxydation s'arrête au stade aldéhyde; on parle alors d'ozonolyse réductrice.

Dans le cas d'une coupure par oxydation avec l'eau oxygénée, on peut adapter les conditions opératoires pour parvenir soit aux fonctions acides en dopant la réaction avec $O_2$, il s'agit là aussi d'une coupure oxydante, soit bloquer la réaction au stade aldéhyde et on parlera d'une coupure par oxydation réductrice. Pour des facilités de langage, on utilisera souvent les termes de coupure oxydante pour celle conduisant à l'acide et de coupure réductrice pour celle conduisant à l'aldéhyde.

En fonction des besoins industriels, on adapte les conditions de la coupure pour obtenir soit l'ester/acide-aldéhyde $R_2$-$(CH_2)_n$-CHO, soit le diacide ou l'ester-acide $R_2$-$(CH_2)_n$-COOH, conduisant par hydrolyse au diacide.

Dans une variante du procédé mettant en oeuvre une ozonolyse réductrice, par décomposition de l'ozonide en présence de $H_2$ par exemple, ou une coupure par $H_2O_2$ dans des conditions « réductrices », on peut poursuivre la réaction avec une réduction de l'aldéhyde pour obtenir une fonction alcool. Cette voie permet d'obtenir un ester/acide-alcool $R_2$-$(CH_2)_n$-$CH_2OH$, monomère de polyesters à chaines longues. Cette approche s'applique tout aussi bien et est même particulièrement pertinente dans le cas de l'ozonolyse réductrice, où l'ozonide intermédiaire est décomposé par l'hydrogène en présence d'un catalyseur (par exemple Pt) pour former l'aldéhyde et il suffit de poursuivre la réduction de l'aldéhyde en alcool pour obtenir le monomère recherché.

L'étape de coupure oxydante cp2 est éventuellement suivie d'au moins une étape optionnelle choisie parmi :

- l'oxydation de l'aldéhyde en présence de dioxygène pour le transformer en acide ;
- l'hydrolyse de la fonction ester pour former un diacide de formule

$$HOOC\text{-}(CH_2)_n\text{-}COOH ;$$

- l'amination réductrice de la fonction aldéhyde en amino-ester/acide ;
- l'hydrogénation de l'aldéhyde-ester/acide en alcool-ester ou alcool-acide respectivement, utilisable dans la synthèse de polyesters ;
- l'ammoniation de l'acide-ester ou du diacide pour sa conversion en dinitrile puis éventuellement hydrogénation en diamine ;
- l'estérification et/ou transestérification de l'acide-ester ou du diacide ;
- et des mélanges de ces étapes. Est également divulgué un procédé de fabrication de composé(s) intermédiaire(s), caractérisé en ce qu'il comprend :
- à partir d'un même composé gras insaturé (I) comportant au moins une fonction respectivement ester ou acide, de formule :

$$R_1\text{-CH=CH-}[(CH_2)_q\text{-CH=CH}]_p\text{ -}(CH_2)_n\text{-}R_2 \qquad \text{(I)}$$

  dans laquelle $R_1$ est H ou un radical alkyle comprenant de 1 à 4 atomes de carbone, $R_2$ est une fonction ester ou acide ; et n, p et q sont des entiers tels que $3 \leq n \leq 13$ ; $0 \leq p \leq 3$ ; $0 \leq q \leq 1$,
- une étape de métathèse croisée mc1 du composé (I) avec un composé nitrile insaturé (II) choisi parmi : l'acrylonitrile, le fumaronitrile, le 2-butènenitrile, le 2-pentènenitrile, le 3-pentène nitrile, le 4-pentènenitrile, dans laquelle mc1 est menée à conversion partielle de façon à obtenir et récupérer séparément au moins les produits suivants : un nitrile-acide(ester) mono-insaturé (III) et un composé symétrique (IV), respectivement diester ou diacide comportant une double liaison située au milieu de la chaîne moléculaire du composé (IV) de formule $R_2\text{-}(CH_2)_n\text{-HC=CH-}(CH_2)_n\text{-}R_2$ résultant de l'homo-métathèse du composé (I).

Avantageusement, le procédé selon l'invention comprend en outre l'hydrogénation d'au moins une partie du composé symétrique (IV) conduisant au composé saturé correspondant (IVs) de formule $R_2\text{-}(CH_2)_n\text{-}CH_2\text{-}CH_2\text{-}(CH_2)_n\text{-}R_2$. Avantageusement, le procédé de synthèse selon l'invention comprend en outre une étape d'hydrogénation du nitrile-ester/acide mono-insaturé (III) obtenu pour former un $\alpha,\omega$-amino-ester/acide saturé.

Est également divulgué un procédé de synthèse de polymère, notamment de polyamide, par polymérisation utilisant au moins un monomère fabriqué selon le procédé de l'invention, comprenant notamment au moins un $\omega$-aminoacide, $\omega$-aminoester, diacide et/ou diester, obtenu selon le procédé de l'invention. Est également divulgué un polymère, notamment polyamide, caractérisé en ce qu'il comprend au moins un monomère, comprenant notamment au moins un $\omega$-aminoacide, $\omega$-aminoester, diacide et/ou diester, fabriqué selon le procédé de l'invention.
De préférence, le polymère, notamment polyamide, est caractérisé en ce qu'il comprend au moins un des monomères de polyamide suivants : 4.6, 4.T, 5.6, 5.9, 5.10, 5.12, 5.13, 5.14, 5.16, 5.18, 5.36, 6, 6.6, 6.9, 6.10, 6.12, 6.13, 6.14, 6.16, 6.18, 6.36, 6 .T, 9, 10.6, 10.9, 10.10, 10.12, 10.13, 10.14, 10.16, 10.18, 10.36, 10.T, 11, 12, 12.6, 12.9, 12.10, 12.12, 12.13, 12.14, 12.16, 12.18, 12.36, 12.T, et leurs mélanges ; et de préférence est choisi parmi le PA 11, le PA 12, le PA 10.10, le PA 6, le PA 6.10, PA 6.12, PA 10.12, PA 6.14 et/ou PA 6.6/6, le PA 11/10.10, et leurs mélanges.

EXEMPLES

Les exemples suivants illustrent l'invention sans la limiter.

Exemple 1 : Métathèse du 9-décénoate de méthyle avec l'acrylonitrile

Le schéma réactionnel de la réaction est le suivant

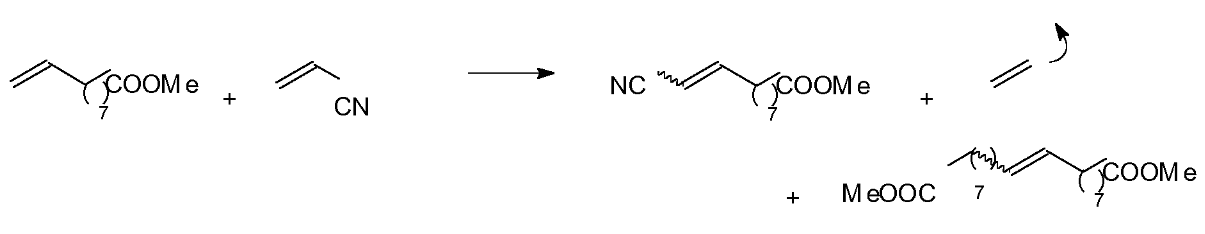

Le catalyseur utilisé pour cette réaction de métathèse est le catalyseur M71-SiPr fourni par la société Umicore de formule (3) :

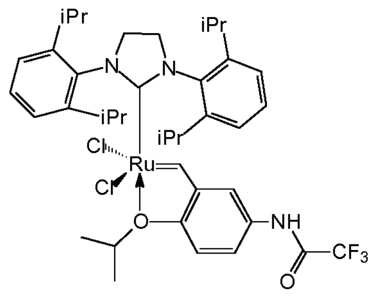

**(3)**

Dans un réacteur en verre de 250 ml muni d'un réfrigérant et purgé à l'azote, on charge 15 g de 9-décénoate de méthyle (81,4 mmol) préparé conformément à l'exemple 1 du document US20110113679, préalablement passé sur colonne d'alumine, 2,15 g d'acrylonitrile (40,7 mmol) et 150 g de toluène séché sur tamis moléculaire. On chauffe à 110°C et on ajoute via des seringues montées sur pousse-seringues, sur une période de 2h, 2,6 g d'acrylonitrile (49 mmol) et 2 mg de catalyseur M71-SiPr ($2{,}44.10^{-6}$ mol) dissous dans 5 g de toluène.

Le mélange réactionnel obtenu est analysé par chromatographie en phase gaz. La conversion du 9-décénoate de méthyle est de 89%. La sélectivité en nitrile-ester insaturé en $C_{11}$ est de 81% et la sélectivité en diester insaturé en $C_{18}$ est de 19%.

La figure 1 est un chromatogramme faisant apparaître à côté des pics correspondant aux nitrile-esters, produits principaux de la réaction de l'exemple 1, le pic caractéristique du 9-octadécènedioate de méthyle (pic à 21,9 min) accompagné d'un pic de très faible amplitude à 20,9, correspondant à un autre diester. La sélectivité relative (9-octadécènedioate de méthyle/diesters totaux de la réaction d'homo-métathèse est supérieure à 98,5 %, calculée sur la base d'un ratio de surface des pics du chromatogramme.

Exemple 2 : Hydrolyse du diester insaturé en $C_{18}$

Le mélange réactionnel obtenu dans l'exemple 1 est évaporé, puis le nitrile-ester en $C_{11}$ est distillé sous vide (température d'ébullition = 121-123°C sous 0,8 mbar).

Le pied de distillation contenant le diester insaturé en $C_{18}$ est récupéré. On charge dans un réacteur 30 g de diester (88 mmol) et 200 g d'eau. On chauffe à 80°C et on ajoute 88 ml de soude 1M (88 mmol). On laisse réagir 1 heure à 80°C. On baisse la température à 50°C et on ajoute une solution aqueuse d'HCl 1M jusqu'à l'obtention d'un pH de 2. Le mélange réactionnel est filtré et le précipité est séché sous vide. On obtient ainsi l'acide 9-octadécènedioïque.

Exemple 3 : Coupure oxydante du diacide insaturé en $C_{18}$

Dans un réacteur double enveloppe de 100 ml, comportant une agitation mécanique et un réfrigérant, 34.9 g d'acide nonanoïque (Aldrich, 97%), 12,8 g acide 9-octadécènedioïque et 2.5 g d'acide tungstique ($H_2WO_4$ ; Merck 98%) sont introduits, puis agités et chauffés à 78°C. La température maintenue par circulation d'eau/glycol thermostatée. 11.1 g de solution aqueuse de peroxyde d'hydrogène (concentration en $H_2O_2$ égale à 70,0 % en poids) est ajoutée goutte à goutte par une pompe péristaltique sur une durée de 6h. Après la première heure un débit d'air de 400 ml/min est injecté dans le mélange réactionnel en utilisant un tube en téflon. La réaction est arrêtée au bout de 24 h.

A la fin de la réaction, la phase organique est lavée plusieurs fois à l'eau déminéralisée jusqu'à disparition du peroxyde d'hydrogène dans l'eau de lavage puis elle est séchée sous vide et analysée par CPG. La conversion est supérieure à 95%. Le rendement en acide azélaïque (diacide en Cg) est de 33%.

Exemple 4 : Métathèse de l'undécénoate de méthyle avec l'acrylonitrile

Le schéma réactionnel est le suivant :

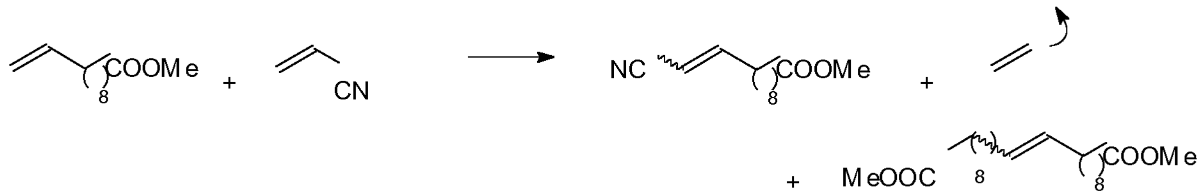

Dans un réacteur en verre de 250 ml muni d'un réfrigérant et purgé à l'azote, on charge 15 g de 10-undécénoate de méthyle (Arkema, 75,6 mmol) préalablement passé sur colonne d'alumine, 2 g d'acrylonitrile (37,7 mmol) et 150 g de toluène séché sur tamis moléculaire. On chauffe à 110°C et on ajoute via des seringues montées sur pousse-seringues, sur une période de 2h, 2,4 g d'acrylonitrile (45,2 mmol) et 1,9 mg de catalyseur M71-SiPr ($2,27.10^{-6}$ mol) dissous dans 5 g de toluène.

Le mélange réactionnel obtenu est analysé par chromatographie en phase gaz. La conversion du 10-undécénoate de méthyle est de 86%. La sélectivité en nitrile-ester insaturé en $C_{11}$ est de 79% et la sélectivité en diester insaturé en $C_{20}$ est de 21%.

Exemple 5 : Hydrolyse du diester insaturé en $C_{20}$

On procède à l'hydrolyse selon le processus décrit dans l'exemple 2 pour obtenir à partir du diester méthylique en C20 issu de l'exemple 4 le diacide insaturé en $C_{20}$ : acide 10-eicosènedioïque.

Exemple 6 : Coupure oxydante du diacide insaturé en $C_{20}$

On procède à la coupure oxydante de l'acide 10-eicosènedioïque selon le processus décrit dans l'exemple 3, mais en utilisant de l'acide butyrique en lieu et place de l'acide nonanoïque. On obtient l'acide sébacique avec un rendement de 35%.

Exemple 7 (non conforme à l'invention) : Homo-métathèse du 9-décénoate de méthyle

Dans un réacteur en verre de 250 ml purgé à l'azote, on introduit 15 g de 9-décénoate de méthyle (81 mmol) préparé selon l'exemple 1 du document de brevet US20110113679 et purifié sur alumine, et 150g de toluène préalablement séché sur tamis moléculaire. On chauffe à 110°C et on introduit via une seringue et un pousse-seringue, sur une période de 2 heures, 2 mg de catalyseur M71-SiPr ($2,4.10^{-3}$ mmol, fourni par la société Umicore) dissous dans 5 ml de toluène. Les conditions opératoires sont identiques à celles de l'exemple 1 exception faite dans le présent exemple de l'absence de composé nitrile insaturé II tel que l'acrylonitrile.

A la fin de l'ajout du catalyseur, le mélange réactionnel est analysé par CPG. La conversion du 9-décénoate de méthyle est supérieure à 99,5%.

L'analyse CPG sur le chromatogramme de la figure 2 montre que la sélectivité en 9-octadécènedioate de méthyle (pic à 21,9 min) ne dépasse pas 68%. D'autres diesters de longueurs de chaîne différentes (pics à 18,6 ; 19,9 ; 20,9 ; 22,7 ; 23,7 ; 24,6) sont formés résultant de réactions d'isomérisation au cours de la réaction de métathèse.

L'application d'une réaction de coupure oxydante à un tel produit de réaction conduirait à côté de l'acide azélaïque à un mélange de quelques 32% d'autres diacides différents et de longueurs de chaîne proches, dont la séparation serait particulièrement difficile.

## Revendications

**1.** Procédé de synthèse conjuguée d'un nitrile-ester(acide) mono-insaturé (III) et d'un composé carbonylé bi-fonctionnel (V) de formule $R_2-(CH_2)_n-COR'$ dans laquelle R' est H ou OH, à partir d'un même composé gras insaturé (I) comportant au moins une fonction respectivement ester ou acide, de formule :

$$R_1-CH=CH-[(CH_2)_q-CH=CH]_p-(CH_2)_n-R_2 \qquad (I)$$

dans laquelle $R_1$ est H ou un radical alkyle comprenant de 1 à 4 atomes de carbone, $R_2$ est une fonction ester ou acide ; et n, p et q sont des entiers tels que $3 \leq n \leq 13$ ; $0 \leq p \leq 3$ ; $0 \leq q \leq 1$,
**caractérisé en ce qu'**il comprend :

- une étape de métathèse croisée mc1 du composé (I) avec un composé nitrile insaturé (II) choisi parmi : l'acrylonitrile, le fumaronitrile, le 2-butènenitrile, le 2-pentènenitrile, le 3-pentène nitrile, le 4-pentènenitrile, dans laquelle mc1 est menée à conversion partielle de façon à obtenir et récupérer séparément au moins les produits suivants : un nitrile-acide(ester) mono-insaturé (III) et un composé symétrique (IV), respectivement diester ou diacide comportant une double liaison située au milieu de la chaîne moléculaire du composé (IV) de formule $R_2$-$(CH_2)_n$-HC=CH-$(CH_2)_n$-$R_2$ résultant de l'homo-métathèse du composé (I), puis
- une étape de coupure par oxydation cp2 de la double liaison du composé (IV), respectivement diester ou diacide, de façon à former un seul type de composé carbonylé (V) de formule $R_2$-$(CH_2)_n$-COR' dans laquelle R' est H ou OH, selon les conditions opératoires choisies pour la coupure par oxydation cp2.

**2.** Procédé selon la revendication 1, dans lequel le composé (I) est un acide gras ou un ester d'un acide gras insaturé d'origine végétale ou animale, ledit acide gras étant choisi de préférence parmi : les acides en C10, les acides obtusilique (cis-4-décénoïque) et caproléique (9-décénoïque), les acides en C12, les acides lauroléique (cis-5-dodécénoïque) et lindérique (cis-4-dodécénoïque), les acides en C14, les acides myristoléique (cis-9-tétradécénoïque), physétérique (cis-5-tetradécénoïque) et tsuzuique (cis-4-tetradécénoïque), l'acide en C16, l'acide palmitoléique (cis-9- hexadécénoïque), les acides en C18, les acides oléique (cis-9-octadécénoïque), élaidique (trans-9-octadécénoïque), pétrosélinique (cis-6-octadécénoïque), vaccénique (cis-11-octadécénoïque) et ricinoléique (12-hydroxy--cis-9-octadécénoïque), les acides en C20, les acides gadoléique (cis-9-eicosénoïque), gondoïque (cis-11-eicosénoïque), l'acide cis-5-eicosènoïque et l'acide lesquérolique (14-hydroxy-cis-11-eicosénoïque), les acides en C22, les acides cétoléique (cis-11-docosénoïque) et érucique (cis-13-docosénoïque) ainsi que les acides polyinsaturés linoléique et linolénique.

**3.** Procédé selon l'une des revendications précédentes **caractérisé en ce qu'**il comprend en outre une étape d'hydrogénation du nitrile-ester/acide mono-insaturé (III) obtenu pour former un $\alpha,\omega$-amino-ester/acide saturé.

**4.** Procédé selon la revendication 3, dans lequel le composé (I) comporte une insaturation localisée en position delta x par rapport au groupement R2 ester ou acide ce qui permet de déterminer la formule de l'$\omega$-amino-ester/acide saturé.

**5.** Procédé selon l'une quelconque des revendications précédentes, **caractérisé en ce qu'**il comprend en outre, préalablement à l'étape mc1, au moins une des réactions suivantes : éthénolyse, buténolyse, propénolyse et/ou craquage thermique (pyrolyse) du composé (I), de façon à obtenir un ester/acide gras de formule :

$$CH_2=CH-(CH_2)_n-R_2,\ CH_3-CH=CH-(CH_2)_n-R_2 \text{ ou } CH_3-CH_2-CH=CH-(CH_2)_n-R_2.$$

**6.** Procédé selon l'une quelconque des revendications précédentes, dans lequel le composé (I) est un ester ou acide gras de formule $CH_2$=CH-$(CH_2)_n$-$COOR_3$ dans laquelle $R_3$ est H ou un radical alkyle comprenant de 1 à 11 atomes de carbone et n est un nombre entier compris dans la gamme de 3 à 13

**7.** Procédé selon l'une quelconque des revendications précédentes, dans lequel le composé nitrile insaturé (II) utilisé lors de l'étape mc1 est l'acrylonitrile.

**8.** Procédé selon l'une quelconque des revendications précédentes, dans lequel l'étape de métathèse croisée est menée à un taux de conversion compris dans la gamme de 30% à 90%, de préférence de 40 à 90%.

**9.** Procédé selon l'une quelconque des revendications précédentes, dans lequel l'étape de métathèse croisée est conduite en présence d'un catalyseur de métathèse de type ruthénium-carbène choisi de préférence parmi les catalyseurs chargés ou non-chargés de formule générale :

$$(X_1)_a\ (X_2)_b\text{Ru(carbène C)}\ (L_1)_c(L_2)_d\ (L_3)_e$$

dans laquelle :

- a, b, c, d et e sont des nombres entiers, identiques ou différents, avec a et b égaux à 0, 1 ou 2 ; c, d et e égaux à 0, 1, 2, 3 ou 4;
- $X_1$ et $X_2$, identiques ou différents, représentent chacun un ligand mono- ou multi-chélatant, chargé ou non tels que les halogénures, le sulfate, le carbonate, les carboxylates, les alcoolates, les phénolates, les amidures, le tosylate, l'hexafluorophosphate, le tétrafluoroborate, le bis-triflylamidure, un alkyle, le tétraphénylborate et

dérivés ; $X_1$ ou $X_2$ peuvent être liés à $L_1$ ou $L_2$ ou au carbène C de façon à former un ligand bidenté ou chélate sur le ruthénium ; et

- $L_1$, $L_2$ et $L_3$ identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéther, ou un carbène hétérocyclique ; $L_1$, $L_2$ ou $L_3$ peuvent être liés au carbène C de façon à former un ligand bidenté ou chélate, ou tridenté.

**10.** Procédé selon la revendication 9 **caractérisé en ce que** le catalyseur répond à la formule :

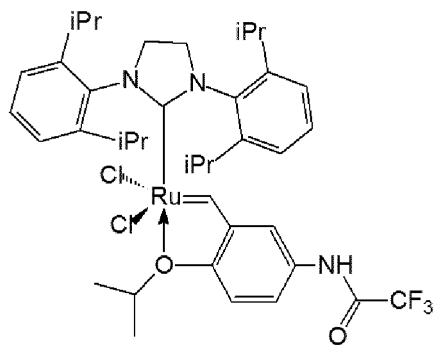

**11.** Procédé selon l'une quelconque des revendications précédentes, **caractérisé en ce qu'**il comprend une étape optionnelle intermédiaire d'hydrolyse entre mc1 et cp2 lorsque le composé (IV) est un diester, pour conduire au composé (IV) diacide.

**12.** Procédé selon l'une quelconque des revendications précédentes, dans lequel l'étape de coupure par oxydation est mise en oeuvre en utilisant comme oxydant l'eau oxygénée et/ou l'ozone.

**13.** Procédé selon l'une des revendications précédentes, **caractérisé en ce que** l'étape de coupure par oxydation est mise en oeuvre dans des conditions oxydantes pour conduire à un composé de formule $R_2$-$(CH_2)_n$-COOH, ester-acide ou diacide.

**14.** Procédé selon la revendication 13 **caractérisé en ce que** le composé de formule $R_2$-$(CH_2)_n$-COOH est soumis à une hydrolyse pour conduire au diacide lorsque $R_2$ est une fonction ester.

**15.** Procédé selon l'une quelconque des revendications précédentes **caractérisé en ce que** l'étape de coupure par oxydation est mise en oeuvre dans des conditions réductrices pour conduire à l'ester/acide-aldéhyde de formule $R_2$-$(CH_2)_n$-CHO.

**16.** Procédé selon la revendication 15 **caractérisé en ce que** le composé de formule $R_2$-$(CH_2)_n$-CHO est soumis à une réduction complémentaire pour conduire à l'ester/acide-alcool de formule $R_2$-$(CH_2)_n$-CH2OH.

**17.** Procédé selon l'une quelconque des revendications 1 à 16, **caractérisé en ce qu'**il comprend en outre l'hydrogénation d'au moins une partie du composé symétrique (IV) conduisant au composé saturé correspondant (IVs) de formule $R_2$-$(CH_2)_n$-$CH_2$-$CH_2$-$(CH_2)_n$-$R_2$.

## Patentansprüche

**1.** Verfahren zur konjugierten Synthese eines einfach ungesättigten Nitrilesters bzw. einer einfach ungesättigten Nitrilsäure (III) und einer bifunktionellen Carbonylverbindung (V) der Formel $R_2$-$(CH_2)_n$-COR', in der R' für H oder OH steht, ausgehend von einer gleichen ungesättigten Fettverbindung (I), die mindestens eine Ester- bzw. Säurefunktion umfasst, der Formel:

$$R_1\text{-CH=CH-[(CH}_2)_q\text{-CH=CH]}_p\text{-(CH}_2)_n\text{-R}_2 \qquad (I),$$

in der $R_1$ für H oder einen Alkylrest mit 1 bis 4 Kohlenstoffatomen steht, $R_2$ für eine Ester- oder Säurefunktion steht

und n, p und q für solche ganzen Zahlen stehen, dass $3 \leq n \leq 13$; $0 \leq p \leq 3$; $0 \leq q \leq 1$,
**dadurch gekennzeichnet, dass** es mindestens Folgendes umfasst:

- einen Schritt der Kreuzmetathese KM1 der Verbindung (I) mit einer ungesättigten Nitrilverbindung (II), die aus Acrylsäurenitril, Fumarsäurenitril, 2-Butennitril, 2-Pentennitril, 3-Pentennitril und 4-Pentennitril ausgewählt wird, wobei KM1 zu teilweisem Umsatz gebracht wird, so dass mindestens die folgenden Produkte erhalten und separat gewonnen werden: eine einfach ungesättigte Nitrilsäure bzw. ein einfach ungesättigter Nitrilester (III) und eine symmetrische Diester- bzw. Disäure-Verbindung (IV) mit einer in der Mitte der Molekülkette der Verbindung (IV) liegenden Doppelbindung der Formel $R_2$-$(CH_2)_n$-HC=CH-$(CH_2)_n$-$R_2$, die sich aus der Homometathese der Verbindung (I) ergibt, dann
- einen Schritt der oxidativen Spaltung OS2 der Doppelbindung der Diester- bzw. Disäure-Verbindung (IV) zur Bildung eines einzigen Typs von Carbonylverbindung (V) der Formel $R_2$-$(CH_2)_n$-COR' (V), in der R' gemäß den für die oxidative Spaltung OS2 gewählten Arbeitsbedingungen für H oder OH steht.

**2.** Verfahren nach Anspruch 1, wobei es sich bei der Verbindung (I) um eine Fettsäure oder einen Ester einer ungesättigten Fettsäure pflanzlicher oder tierischer Herkunft handelt, wobei die Fettsäure vorzugsweise aus: den C10-Säuren Obtusilinsäure (cis-4-Decensäure) und Caproleinsäure (9-Decensäure), den C12-Säuren Lauroleinsäure (cis-5-Dodecensäure) und Linderinsäure (cis-4-Dodecensäure), den C14-Säuren Myristoleinsäure (cis-9-Tetradecensäure), Physeterinsäure (cis-5-Tetradecensäure) und Tsuzuinsäure (cis-4-Tetradecensäure), der C16-Säure Palmitoleinsäure (cis-9-Hexadecensäure), den C18-Säuren Ölsäure (cis-9-Octadecensäure), Elaidinsäure (trans-9-Octadecensäure), Petroselinsäure (cis-6-Octadecensäure), Vaccensäure (cis-11-Octadecensäure) und Ricinoleinsäure (12-Hydroxy-cis-9-octadecensäure), den C20-Säuren Gadoleinsäure (cis-9-Eicosensäure), Gondosäure (cis-11-Eicosensäure), cis-5-Eicosensäure und Lesquerolsäure (14-Hydroxy-cis-11-eicosensäure), den C22-Säuren Cetoleinsäure (cis-11-Docosensäure) und Erucasäure (cis-13-Docosensäure) sowie den mehrfach ungesättigten Säuren Linolsäure und Linolensäure, ausgewählt wird.

**3.** Verfahren nach einem der vorhergehenden Ansprüche, **dadurch gekennzeichnet, dass** es außerdem einen Schritt der Hydrierung des erhaltenen einfach ungesättigten Nitrilesters bzw. der erhaltenen einfach ungesättigten Nitrilsäure (III) zur Bildung eines gesättigten $\alpha,\omega$-Aminoesters bzw. einer gesättigten $\alpha,\omega$-Aminosäure umfasst.

**4.** Verfahren nach Anspruch 3, wobei die Verbindung (I) eine Ungesättigtheit in der Delta-x-Position zur Ester- bzw. Säuregruppe R2 enthält, was es ermöglicht, die Formel des $\omega$-Aminoesters bzw. der $\omega$-Aminosäure zu bestimmen.

**5.** Verfahren nach einem der vorhergehenden Ansprüche, **dadurch gekennzeichnet, dass** es außerdem vor dem Schritt KM1 mindestens eine der folgenden Umsetzungen umfasst: Ethenolyse, Butenolyse, Propenolyse und/oder thermisches Cracken (Pyrolyse) der Verbindung (I) zum Erhalt eines Fettesters bzw. einer Fettsäure der Formel: $CH_2$=CH-$(CH_2)_n$-$R_2$, $CH_3$-CH=CH-$(CH_2)_n$-$R_2$ bzw. $CH_3$-$CH_2$-CH=CH-$(CH_2)_n$-$R_2$.

**6.** Verfahren nach einem der vorhergehenden Ansprüche, wobei es sich bei der Verbindung der Formel (I) um einen Fettester bzw. eine Fettsäure der Formel $CH_2$=CH-$(CH_2)_n$-$COOR_3$, in der $R_3$ für H oder einen Alkylrest mit 1 bis 11 Kohlenstoffatomen steht und n für eine ganze Zahl im Bereich von 3 bis 13 steht, handelt.

**7.** Verfahren nach einem der vorhergehenden Ansprüche, wobei es sich bei der in Schritt KM1 verwendeten ungesättigten Nitrilverbindung (II) um Acrylsäurenitril handelt.

**8.** Verfahren nach einem der vorhergehenden Ansprüche, wobei der Schritt der Kreuzmetathese bis zu einem Umsatzgrad im Bereich von 30 bis 90 %, vorzugsweise 40 bis 90 %, gebracht wird.

**9.** Verfahren nach einem der vorhergehenden Ansprüche, wobei der Schritt der Kreuzmetathese in Gegenwart eines Metathesekatalysators vom Ruthenium-Carben-Typ durchgeführt wird, der vorzugsweise aus den geladenen oder ungeladenen Katalysatoren der allgemeinen Formel:

$$(X_1)_a(X_2)_b\text{Ru (Carben C) }(L_1)_c(L_2)_d(L_3)_e$$

ausgewählt wird, wobei:

- a, b, c, d und e für gleiche oder verschiedene ganze Zahlen stehen, wobei a und b gleich 0, 1 oder 2 sind; c, d und e gleich 0, 1, 2, 3 oder 4 sind;

- $X_1$ und $X_2$ gleich oder verschieden sind und jeweils für einen geladenen oder ungeladenen Mono- oder Multichelatliganden wie Halogenide, Sulfat, Carbonat, Carboxylate, Alkoholate, Phenolate, Amide, Tosylat, Hexafluorophosphat, Tetrafluoroborat, Bis(triflyl)amid, ein Alkyl, Tetraphenylborat und Derivate stehen; $X_1$ oder $X_2$ an $L_1$ oder $L_2$ oder an das Carben C gebunden sein können, so dass sich ein zweizähniger Ligand oder Chelatligand am Ruthenium ergibt; und
- $L_1$, $L_2$ und $L_3$ gleich oder verschieden sind und für elektronenliefernde Liganden wie Phosphin, Phosphit, Phosphonit, Phosphinit, Arsin, Stilbin, ein Olefin oder einen Aromaten, eine Carbonylverbindung, einen Ether, einen Alkohol, ein Amin, ein Pyridin oder Derivat, ein Imin, einen Thioether oder ein heterocyclisches Carben stehen; $L_1$, $L_2$ und $L_3$ an das Carben C gebunden sein können, so dass sich ein zweizähniger Ligand oder Chelatligand oder ein dreizähniger Ligand ergibt.

**10.** Verfahren nach Anspruch 9, **dadurch gekennzeichnet, dass** der Katalysator der Formel:

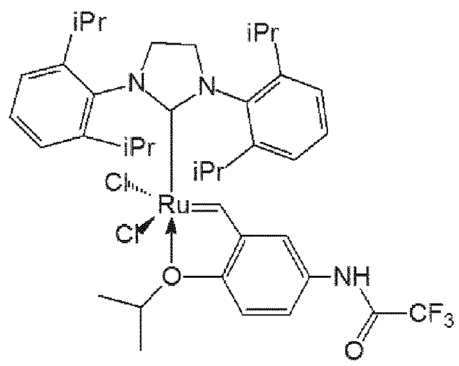

entspricht.

**11.** Verfahren nach einem der vorhergehenden Ansprüche, **dadurch gekennzeichnet, dass** es dann, wenn es sich bei der Verbindung (IV) um einen Diester handelt, einen fakultativen Hydrolyse-Zwischenschritt zwischen KM1 und OS2 umfasst, was zu der Disäure-verbindung (IV) führt.

**12.** Verfahren nach einem der vorhergehenden Ansprüche, wobei der Schritt der oxidativen Spaltung unter Verwendung von wässriger Wasserstoffperoxidlösung und/oder Ozon als Oxidationsmittel durchgeführt wird.

**13.** Verfahren nach einem der vorhergehenden Ansprüche, **dadurch gekennzeichnet, dass** der Schritt der Oxepinspaltung oder oxidierenden Bedingungen durchgeführt wird, was zu einer Estersäure- bzw. Disäureverbindung der Formel $R_2$-$(CH_2)_n$-COOH führt.

**14.** Verfahren nach Anspruch 13, **dadurch gekennzeichnet, dass** dann, wenn $R_2$ für eine Esterfunktion steht, die Verbindung der Formel $R_2$-$(CH_2)_n$-COOH einer Hydrolyse unterworfen wird, was zu der Disäure führt.

**15.** Verfahren nach einem der vorhergehenden Ansprüche, **dadurch gekennzeichnet, dass** der Schritt der oxidativen Spaltung unter reduzierenden Bedingungen durchgeführt wird, was zu der Estersäure bzw. dem Esteraldehyd der Formel $R_2$-$(CH_2)_n$-CHO führt.

**16.** Verfahren nach Anspruch 15, **dadurch gekennzeichnet, dass** die Verbindung der Formel $R_2$-$(CH_2)_n$-CHO einer zusätzlichen Reduktion unterworfen wird, was zudem Esteralkohol bzw. dem Säurealkohol der Formel $R_2$-$(CH_2)_n$-$CH_2OH$ führt.

**17.** Verfahren nach einem der Ansprüche 1 bis 16, **dadurch gekennzeichnet, dass** es außerdem die Hydrierung mindestens eines Teils der symmetrischen Verbindung (IV) umfasst, was zu der entsprechenden gesättigten Verbindung (IVg) der Formel $R_2$-$(CH_2)_n$-$CH_2$-$CH_2$-$(CH_2)_n$-$R_2$ führt.

## Claims

**1.** Process for the conjugate synthesis of a monounsaturated nitrile ester (acid) (III) and of a difunctional carbonyl

compound (V), of formula $R_2$-$(CH_2)_n$-COR' in which R' is H or OH, from the same unsaturated fatty compound (I) comprising at least one ester or acid function, respectively, of formula:

$$R_1\text{-CH=CH-[(CH}_2)_q\text{-CH=CH]}_p\text{-(CH}_2)_n\text{-R}_2 \qquad (I)$$

in which $R_1$ is H or an alkyl radical comprising from 1 to 4 carbon atoms, $R_2$ is an ester or acid function and n, p and q are integers such that $3 \leq n \leq 13$; $0 \leq p \leq 3$; $0 \leq q \leq 1$,
**characterized in that** it comprises:

- a step of cross metathesis cml of compound (I) with an unsaturated nitrile compound (II) chosen from: acrylonitrile, fumaronitrile, 2-butenenitrile, 2-pentenenitrile, 3-pentenenitrile and 4-pentenenitrile, in which cm1 is brought to partial conversion so as to obtain, and to recover separately, at least the following products: a monounsaturated acid (ester)-nitrile (III) and a symmetrical compound (IV), respectively a diester or diacid comprising a double bond located in the middle of the molecular chain of compound (IV) of formula $R_2$-$(CH_2)_n$-HC=CH-$(CH_2)_n$-$R_2$ resulting from the homometathesis of compound (I), and then
- a step of oxidative cleavage c12 of the double bond respectively of the diester or diacid compound (IV), so as to form a single type of carbonyl compound (V) of formula $R_2$-$(CH_2)_n$-COR' in which R' is H or OH, depending on the operating conditions chosen for the oxidative cleavage cl2.

**2.** Process according to Claim 1, in which compound (I) is a fatty acid or an ester of an unsaturated fatty acid of plant or animal origin, said fatty acid preferably being chosen from: C10 acids, obtusilic acid (cis-4-decenoic acid) and caproleic acid (9-decenoic acid), C12 acids, lauroleic acid (cis-5-dodecenoic acid) and linderic acid (cis-4-dodecenoic acid), C14 acids, myristoleic acid (cis-9-tetradecenoic acid), physeteric acid (cis-5-tetradecenoic acid) and tsuzuic acid (cis-4-tetradecenoic acid), C16 acids, palmitoleic acid (cis-9-hexadecenoic acid), C18 acids, oleic acid (cis-9-octadecenoic acid), elaidic acid (trans-9-octadecenoic acid), petroselinic acid (cis-6-octadecenoic acid), vaccenic acid (cis-11-octadecenoic acid) and ricinoleic acid (12-hydroxy-cis-9-octadecenoic acid), C20 acids, gadoleic acid (cis-9-eicosenoic acid), gondoic acid (cis-11-eicosenoic acid), cis-5-eicosenoic acid and lesquerolic acid (14-hydroxy-cis-11-eicosenoic acid), C22 acids, cetoleic acid (cis-11-docosenoic acid) and erucic acid (cis-13-docosenoic acid), and also the polyunsaturated acids linoleic acid and linolenic acid.

**3.** Process according to either of the preceding claims, **characterized in that** it also comprises a step of hydrogenation of the monounsaturated ester/acid nitrile (III) obtained to form a saturated $\alpha,\omega$-amino ester/acid.

**4.** Process according to Claim 3, in which compound (I) comprises an unsaturation located in the delta x position relative to the ester or acid group R2, which makes it possible to determine the formula of the saturated $\omega$-amino ester/acid.

**5.** Process according to any one of the preceding claims, **characterized in that** it also comprises, prior to step cml, at least one of the following reactions: ethenolysis, butenolysis, propenolysis and/or thermal cracking (pyrolysis) of compound (I), so as to obtain a fatty ester/acid of formula:

$$CH_2\text{=CH-(CH}_2)_n\text{-R}_2,\ CH_3\text{-CH=CH-(CH}_2)_n\text{-R}_2 \text{ or } CH_3\text{-CH}_2\text{-CH=CH-(CH}_2)_n\text{-R}_2.$$

**6.** Process according to one of the preceding claims, in which compound (I) is a fatty ester or acid of formula $CH_2$=CH-$(CH_2)_n$-$COOR_3$ in which $R_3$ is H or an alkyl radical comprising from 1 to 11 carbon atoms and n is an integer in the range from 3 to 13.

**7.** Process according to any one of the preceding claims, in which the unsaturated nitrile compound (II) used in step cml is acrylonitrile.

**8.** Process according to any one of the preceding claims, in which the cross metathesis step is performed to a degree of conversion in the range from 30% to 90%, preferably from 40% to 90%.

**9.** Process according to any one of the preceding claims, in which the cross metathesis step is performed in the presence of a metathesis catalyst of ruthenium-carbene type preferably chosen from the charged or uncharged catalysts of general formula:

$$(X_1)_a (X_2)_b\text{Ru (carbene C) } (L_1)_c(L_2)_d(L_3)_e$$

in which:

- a, b, c, d and e are integers, which may be identical or different, with a and b equal to 0, 1 or 2; c, d and e equal to 0, 1, 2, 3 or 4;
- $X_1$ and $X_2$, which may be identical or different, each represent a charged or uncharged and monochelating or polychelating ligand such as halides, sulfate, carbonate, carboxylates, alkoxides, phenoxides, amides, tosylate, hexafluorophosphate, tetrafluoroborate, bis(triflyl)amide, an alkyl, tetraphenylborate and derivatives; $X_1$ or $X_2$ can be bonded to $L_1$ or $L_2$ or to the carbene C so as to form a bidentate or chelate ligand on the ruthenium; and
- $L_1$, $L_2$ and $L_3$, which may be identical or different, are electron-donating ligands, such as phosphine, phosphite, phosphonite, phosphinite, arsine, stilbene, an olefin or an aromatic compound, a carbonyl compound, an ether, an alcohol, an amine, a pyridine or derivative, an imine, a thioether, or a heterocyclic carbene; $L_1$, $L_2$ or $L_3$ can be bonded to the carbene C so as to form a bidentate or chelate ligand, or a tridentate ligand.

**10.** Process according to Claim 9, **characterized in that** the catalyst corresponds to the formula:

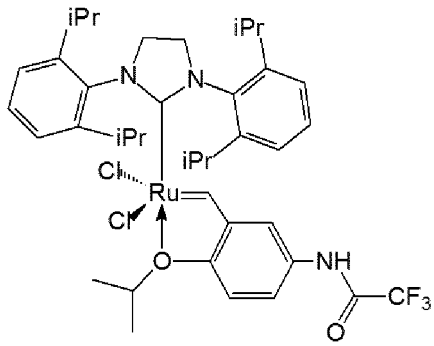

**11.** Process according to any one of the preceding claims, **characterized in that** it comprises an optional intermediate step of hydrolysis between cml and c12 when compound (IV) is a diester, to give the diacid compound (IV) .

**12.** Process according to any one of the preceding claims, in which the oxidative cleavage step is performed using aqueous hydrogen peroxide solution and/or ozone as oxidizing agent.

**13.** Process according to one of the preceding claims, **characterized in that** the oxidative cleavage step is performed under oxidative conditions to give a compound of formula $R_2$-$(CH_2)_n$-COOH, acid ester or diacid.

**14.** Process according to Claim 13, **characterized in that** the compound of formula $R_2$-$(CH_2)_n$-COOH is subjected to a hydrolysis to give the diacid when $R_2$ is an ester function.

**15.** Process according to any one of the preceding claims, **characterized in that** the oxidative cleavage step is performed under reductive conditions to give the ester/acid-aldehyde of formula $R_2$-$(CH_2)_n$-CHO.

**16.** Process according to Claim 15, **characterized in that** the compound of formula $R_2$-$(CH_2)_n$-CHO is subjected to an additional reduction to give the ester/acid-alcohol of formula $R_2$-$(CH_2)_n$-CH2OH.

**17.** Process according to any one of Claims 1 to 16, **characterized in that** it also comprises the hydrogenation of at least part of the symmetrical compound (IV) leading to the corresponding saturated compound (IVs) of formula $R_2$-$(CH_2)_n$-$CH_2$-$CH_2$-$(CH_2)_n$-$R_2$.

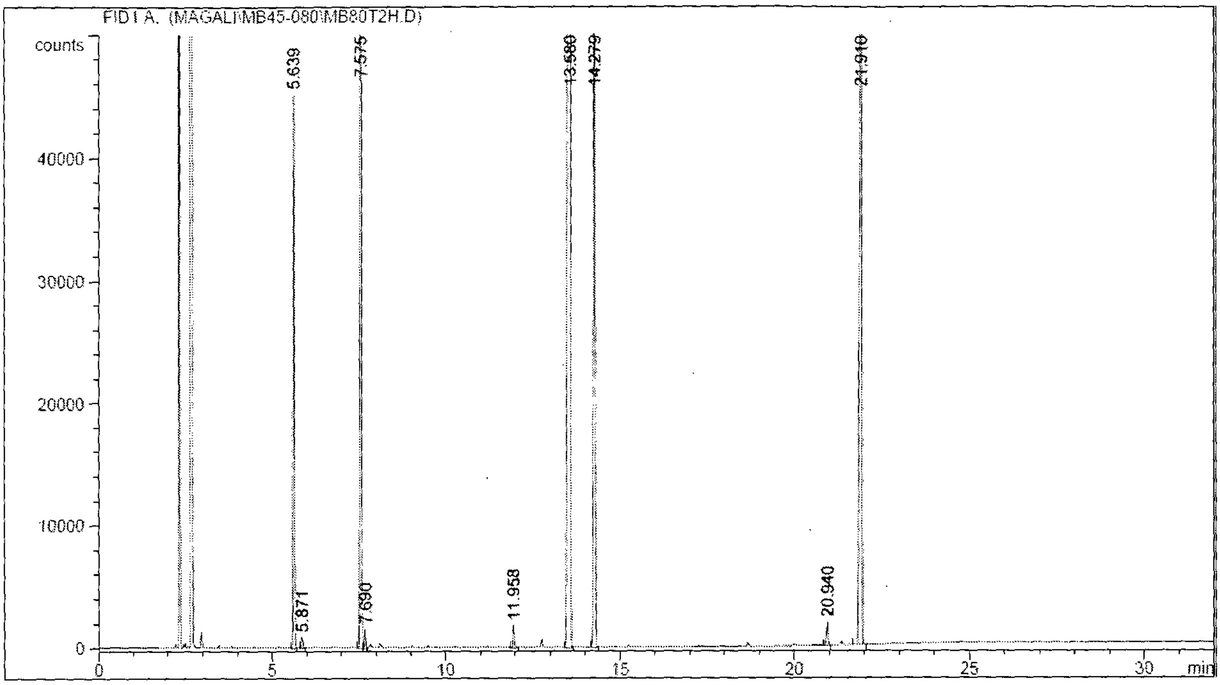
FID1 A, (MAGALI\MB45-080\MB80T2H.D)
counts
40000
30000
20000
10000
0
5.639
7.575
13.580
14.279
21.910
5.871
7.690
11.958
20.940
0
5
10
15
20
25
30
min

**Figure 1**

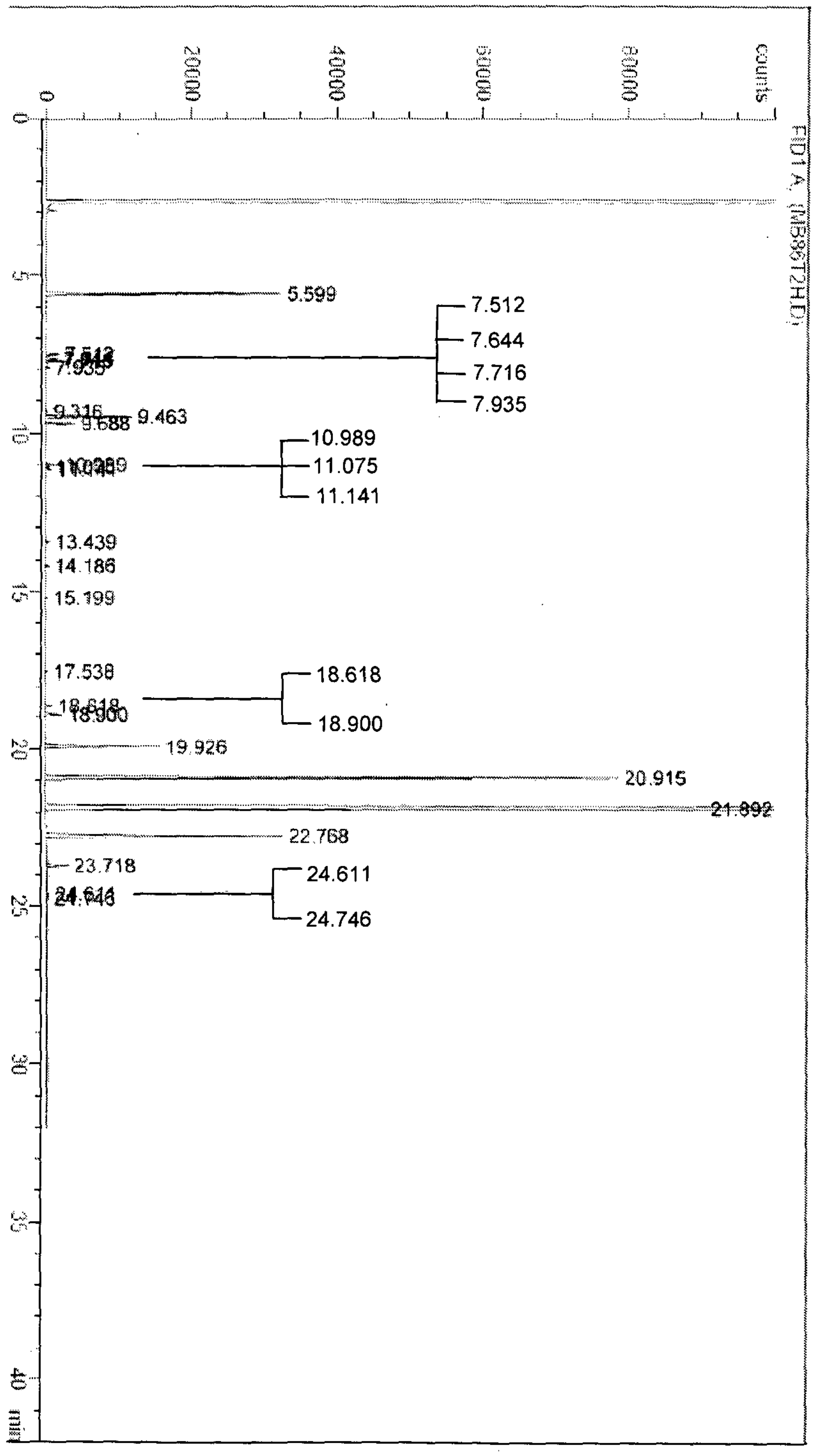

FID1 A, (MB86T2H.D)
counts
0
20000
40000
60000
80000
min
5.599
7.512
7.644
7.716
7.935
9.316
9.463
9.688
10.989
11.075
11.141
13.439
14.186
15.199
17.538
18.618
18.900
19.926
20.915
21.892
22.768
23.718
24.611
24.746


Figure 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- GB 741739 A
- WO 08104722 A
- WO 10055273 A
- WO 10089512 A
- WO 08155506 A
- WO 09047444 A
- GB 743491 A
- US 2813113 A
- US 20110113679 A


### Littérature non-brevet citée dans la description


- **A. CHAUVEL et al.** Les Procédés de Pétrochimie. 1986
- n-Nylons, Their Synthesis, Structure and Properties. 1997, 381-389
- **A. RAVVE.** Organic Chemistry of Macromolecules. Marcel Dekker, Inc, 1967
- **E. H. PRYDE et al.** Aldehydic Materials by the Ozonization of Vegetable Oils. *Journal of the American Oil Chemists Society,* 1962, vol. 39, 496-500
- *Pilot Run, Plant Design and Cost Analysis for Reductive Ozonolysis of Methyl Soyate,* vol. 49, 643-648
- **R.B. PERKINS et al.** Nylon-9 from Unsaturated Fatty Derivatives : Preparation and Characterization. *JAOCS,* vol. 52, 473-477
- Encyclopédie Kirk-Othmer. vol. A8, 118-136
- **SCHROCK et al.** *J. Am. Chem. Soc.,* 1986, vol. 108, 2771
- **BASSET et al.** *Angew. Chem., Ed. Engl.,* 1992, vol. 31, 628
- **GRUBBS et al.** Ed. Engl. *Angew. Chem.,* 1995, vol. 34, 2039
- *Organic Letters,* 1999, vol. 1, 953
- *Aldrichimica Acta,* 2007, vol. 40 (2), 45-52
- Reactions of Alkenes. **L.G. WADE JR.** Organic Chemistry
- **G.S. ZHANG et al.** *Chinese Chemical Letters,* 1994, vol. 5 (2), 105-108
- **F. DRAWERT et al.** *Chem. Mikrobiol. Technol. Lebensm.,* 1972, vol. 1, 158-159